# EUROPÄISCHE PATENTSCHRIFT

(11) **EP 4 043 004 B1**
(45) Veröffentlichungstag und Bekanntmachung des Hinweises auf die Patenterteilung: **20.08.2025**
(21) Anmeldenummer: 22153751.7
(22) Anmeldetag: 27.01.2022
(51) Int. Cl.: A61K 8/34, A61K 8/37, A61Q 17/00, A61Q 19/00

(54) **HYGIENEGEL MIT HAUTPFLEGENDEM EFFEKT UND ANTIMIKROBIELLER WIRKSAMKEIT**
HYGIENIC GEL WITH SKIN CARE EFFECT AND ANTIMICROBIAL EFFECT
GEL D'HYGIÈNE AYANT UN EFFET DE SOIN DERMATOLOGIQUE ET UNE ACTIVITÉ ANTIMICROBIENNE

(30) Priorität: 29.01.2021 DE 102021102173
(43) Veröffentlichungstag der Anmeldung: 17.08.2022
(73) Patentinhaber: Dr. Schumacher GmbH, 34323 Malsfeld-Beiseförth (DE)
(72) Erfinder: Pogrzeba, Jessica, 34323 Malsfeld (DE); Herrlich, Luisa, 34323 Malsfeld (DE); Hillmann, Hannah, 34323 Malsfeld (DE); Ballez, Mike, 34323 Malsfeld (DE)
(74) Vertreter: Sandvoß, Stefanie

(56) Entgegenhaltungen:
- EP-A2- 0 547 727
- EP-B2- 1 685 854
- WO-A1-2014/201541
- DE-A1- 102018 120 717

## Beschreibung

Die Erfindung betrifft ein Hygienegel mit hautpflegendem Effekt und antimikrobieller Wirksamkeit. Weiterhin betrifft die Erfindung auch ein Verfahren zur Reinigung von Haut sowie die Verwendung des Hygienegels zur Reinigung von Haut.

Gerade in der heutigen Zeit, in der unser Leben in so vielen Bereichen von der COVID-19-Pandemie geprägt ist, ist es für einen Großteil der Bevölkerung zur Gewohnheit geworden, mehrmals täglich, beispielsweise nach dem Besuch öffentlicher Einrichtungen, dem Einkaufen im Supermarkt oder der Fahrt mit öffentlichen Verkehrsmitteln, die Hände mit Desinfektionsmittel zu desinfizieren. Desinfektionsmittel beseitigen wirksam Krankheitserreger auf der Haut und auch auf Flächen und minimieren dadurch das Risiko einer Infektion. Allerdings bringt eine häufige bzw. regelmäßige Anwendung von Desinfektionsmitteln auf der Haut auch den Nachteil mit sich, dass die natürliche Schutzschicht der Haut angegriffen wird.

Insofern ist es wichtig, die durch Desinfektionsmittel angegriffene Haut durch regelmäßige Verwendung hautpflegender Produkte zu pflegen und somit eine geschädigte Schutzschicht wieder aufzubauen.

Um die Haut zusätzlich zu schonen, ist es überdies wichtig, Desinfektionsmittel nur dann zu verwenden, wenn deren Einsatz auch wirklich notwendig bzw. sinnvoll ist. So ist es beispielsweise im privaten Bereich, z.B. beim Wickeln eines Kindes, natürlich wichtig, dass die Hände möglichst frei von pathogenen Keimen sind. Für die Verwendung von harten Desinfektionsmitteln besteht jedoch keine Indikation, diese wäre unverhältnismäßig, da die Haut, insbesondere bei regelmäßiger Anwendung, unnötig geschädigt würde.

Die EP 1 056 338 A1 beschreibt Desinfektionsgele mit geringem Alkoholgehalt, die zusätzlich biozide Wirkstoffe wie z.B. Triclosan (Irgasan^{®} DP-300 (Ciba)), Phenoxyethanol, Chlorhexidin-Gluconat, Povidon-Iod, lodophor, Benzalkoniumchlorid, Benzethoniumchlorid und Kresol enthalten.

Die CN111050552 A beschreibt antimikrobielle Zusammensetzungen, die 18 Gew.-% Alkohol sowie organische Säuren und Parabene enthalten, die jedoch heutzutage nicht mehr erwünscht sind.

Die DE 10 2018 120 717 A1 beschreibt eine Zusammensetzung mit desinfizierender Wirkung, die mindestens 45 Gew.-% Alkohol, mind. 0,3 Gew.-% Feuchthaltemittel und vorzugsweise ein Rückfettersystem aus Isopropylpalmitate, Myristylalkohol und Octyldodecanol enthält.

Weitere Desinfektionsmittel mit noch deutlich höherem Alkoholgehalt werden in der DE 10 2016 113 210 A1, in der EP 3 205 211 A1 sowie in der US 4,956,170 offenbart.

Die EP 1 685 854 B2 beschreibt ein viruzides Handdesinfektionsmittel, das in flüssiger oder bevorzugt in gelförmiger Form vorliegen kann und z.B. 52,4 Gew.-% Ethanol, 21,0 Gew.-% Propanol sowie Octyldodecanol und Glycerin enthält.

Die EP 0 547 727 A2 beschreibt Handdesinfektionsmittel mit Hautpflegekomponenten, die ein angenehmes Hautgefühl vermitteln sollen. Die Zusammensetzungen enthalten jeweils Glycerinmonoalkylether, der in Kombination mit ebenfalls enthaltenem Alkohol eine synergistische rückfettende Wirkung zeigen soll.

Die WO 2014/201541 beschreibt flüssige Zusammensetzungen ohne antimikrobielle Wirksamkeit mit einem Alkoholanteil von < 35 Gew.-%, die bei Betätigung einer Pumpe Schaum produzieren sollen und deshalb neben einem Lösungsvermittler ein Schaummittel und einen Schaumverstärker, jedoch kein Treibmittel, enthalten. Optional enthalten die Zusammensetzungen auch ein Rückfettungsmittel.

### Aufgabe der Erfindung

Es ist die Aufgabe der vorliegenden Erfindung, ein alternatives biozidfreies Hygienegel, insbesondere für die Anwendung im privaten Bereich bereitzustellen, das sich durch einen hautpflegenden Effekt und eine gute antimikrobielle Wirksamkeit auszeichnet, die sich bevorzugt gegen Viren, Bakterien und Pilze richtet.

### Allgemeine Beschreibung der Erfindung

Die Erfindung löst diese Aufgabe mit den Merkmalen der Ansprüche und insbesondere mit einem Hygienegel mit hautpflegendem Effekt und antimikrobieller Wirksamkeit, das die folgenden Inhaltsstoffe enthält:
- 5 bis 35 Gew.-% Alkohol, wobei der Alkohol 1 bis 4 Kohlenstoffatome aufweist;
- zumindest 0,3 Gew.-% Feuchthaltemittel, wobei das Feuchthaltemittel aus der Gruppe ausgewählt ist, die Glycerin, Propylenglycol, Butan-1,2-diol, Butan-1,3-diol und Kombinationen dieser umfasst; und
- ein Rückfettersystem, das Isopropylpalmitate, Myristylalkohol und 2-Octyldodecanol als Verbindungen mit rückfettenden Eigenschaften enthält.

Unter einem "Hygienegel" wird für die Zwecke der Erfindung eine flüssige Zusammensetzung mit einer Viskosität von 115 mPa·s bis 450 mPa·s, gemessen mit einem DIN-Messsystem 33 gemäß ISO 53019 bei einer Temperatur von 293,15 K und mit einer Umdrehungszahl von 500 r/min, verstanden, wobei die Zusammensetzung eine hygienische Wirksamkeit, insbesondere eine antimikrobielle Wirksamkeit, aufweist.

Unter einer antimikrobiellen Wirksamkeit wird insbesondere eine Wirksamkeit gegen Viren, Bakterien und/oder Pilze verstanden.

Erfindungsgemäß weist das Hygienegel eine Viskosität von 115 mPa·s bis 450 mPa·s auf, vorzugsweise von 200 mPa·s bis 400 mPa·s und besonders bevorzugt von 250 mPa·s bis 350 mPa·s, jeweils gemessen mit einem DIN-Messsystem 33 gemäß ISO 53019 bei einer Temperatur von 293,15 K und mit einer Umdrehungszahl von 500 r/min. Aufgrund seiner gelartigen Konsistenz ist das Hygienegel besonders angenehm in der Anwendung, denn es kann problemlos auf die Haut, insbesondere die Hände, aufgetragen und dort ohne Zusatz von Wasser oder Tüchern bis zur Trocknungszeit verrieben werden. Anders als Desinfektionsmittel, die eine geringere Viskosität aufweisen und bei denen bei der Anwendung oftmals ein kleiner Anteil verloren geht, da das niedrigviskose Mittel leicht von den Händen rinnt, verbleibt das erfindungsgemäße Hygienegel aufgrund seiner höheren Viskosität selbst bei geneigter Handhaltung auf der Hautoberfläche, sodass das gesamte auf die Hautoberfläche applizierte Hygienegel ohne Verlust zur Reinigung der Hände genutzt werden kann.

Im Gegensatz zu herkömmlichen Desinfektionsmitteln zeichnet sich das erfindungsgemäße Hygienegel darüber hinaus durch seinen geringen Alkoholgehalt aus, der signifikant dazu beiträgt, dass die Haut selbst bei regelmäßiger Anwendung nicht übermäßig austrocknet. Während andere aus dem Stand der Technik bekannte Zusammensetzungen, die ebenfalls einen niedrigen Alkoholgehalt aufweisen, eine hinreichende Wirksamkeit nur bei Zusatz biozid wirkender Inhaltsstoffe erreichen, zeigte sich überraschend, dass ein vollkommen biozidfreies Hygienegel mit erfindungsgemäß geringem Alkoholgehalt in Kombination mit einem Rückfettersystem aus Isopropylpalmitate, Myristylalkohol und 2-Octyldodecanol und zumindest einem Feuchthaltemittel sowohl hervorragende hautpflegende Eigenschaften als auch eine gute antimikrobielle Wirksamkeit aufweist. Die antimikrobielle Wirksamkeit äußert sich dabei in einer sehr guten Keimzahlreduktion, die zwar nicht einer von klassischen Desinfektionsmitteln erreichten Keimzahlreduktion entspricht, allerdings für Bereiche, in denen dies auch gar nicht erforderlich ist, insbesondere im privaten Bereich, völlig ausreichend ist. Die Anwendung des erfindungsgemäßen Hygienegels, das vorzugsweise beim Verreiben auf der Haut eine Trocknungszeit von wenigen Sekunden aufweist, ermöglicht daher eine hygienische Reinigung und gleichzeitige Pflege der Haut, auf die es appliziert wird.

Der in dem erfindungsgemäßen Hygienegel enthaltene Alkohol ist vorzugsweise ausgewählt aus der Gruppe, die Methanol, Ethanol, Propanol, Isopropanol, Butanol, Isobutanol und Mischungen dieser umfasst. Der Anteil an Alkohol an dem Hygienegel beträgt dabei erfindungsgemäß 5 bis 35 Gew.-%, insbesondere 10 bis 30 Gew.-%, bevorzugt 10 bis 25 Gew.-% und besonders bevorzugt 10 bis 20 Gew.-%.

Unter einem Feuchthaltemittel werden für die Zwecke der Erfindung Verbindungen verstanden, die die Feuchtigkeit der Haut erhöhen. Dabei sind mindestens 0,3 Gew.-% Feuchthaltemittel in dem erfindungsgemäßen Hygienegel enthalten. Insbesondere beträgt der Anteil an Feuchthaltemittel in dem Hygienegel 0,3 bis 3,5 Gew.-%, bevorzugt 0,4 bis 3,0 Gew.-%, bevorzugter 0,5 bis 2,0 Gew.-% und besonders bevorzugt 0,5 bis 1,2 Gew.-%. Es können eine oder mehrere Verbindungen in beliebiger Kombination aus der Gruppe, die Glycerin, Propylenglycol, Butan-1,2-diol und Butan-1,3-diol umfasst, als Feuchthaltemittel in dem erfindungsgemäßen Hygienegel enthalten sein.

Das erfindungsgemäße Hygienegel enthält weiterhin ein Rückfettersystem, das Isopropylpalmitate, Myristylalkohol und 2-Octyldodecanol als Verbindungen mit rückfettenden Eigenschaften enthält. Unter einem "Rückfettersystem" wird dabei eine Kombination von verschiedenen Verbindungen mit rückfettenden Eigenschaften verstanden, wobei unter einer Verbindung mit rückfettenden Eigenschaften für die Zwecke der Erfindung eine Verbindung verstanden wird, die dem entfettenden Charakter einer Formulierung entgegenwirkt.

Das vorgenannte Rückfettersystem aus Isopropylpalmitate, Myristylalkohol und 2-Octyldodecanol hat sich als optimal herausgestellt, um einem Hygienegel, das zusätzlich ein Feuchthaltemittel enthält, auch bei vergleichsweise geringem Alkoholgehalt eine gute antimikrobielle Wirksamkeit auf der einen Seite und einen guten hautpflegenden Effekt auf der anderen Seite zu verleihen.

Optional kann das erfindungsgemäße Hygienegel neben dem vorgenannten Rückfettersystem noch eine oder mehrere weitere Verbindungen mit rückfettenden Eigenschaften enthalten, die aus der Gruppe ausgewählt ist bzw. sind, die PEG-7 Glyceryl Cocoate, Dicaprylyl Ether, Lanolin, Sorbitan Sesquicaprylate, Isopropylmyristate, Polyglyceryl-4-Caprate, Capryl/Caprinsäure Triglycerid (Caprylic/Capric Triglyceride), Cetearyl Ethylhexanoate, Glyceryl Laurate und Kombinationen dieser umfasst oder daraus besteht.

Der Gesamtanteil des Rückfettersystems aus Isopropylpalmitate, Myristylalkohol und 2-Octyldodecanol und optional weiteren Verbindungen mit rückfettenden Eigenschaften an dem erfindungsgemäßen Hygienegel beträgt vorzugsweise 0,3 bis 10,0 Gew.-%, insbesondere 0,5 bis 5,0 Gew.-% und besonders bevorzugt 2,0 bis 3,5 Gew.-%.

In der besonders bevorzugten Ausführungsform, in der der Gesamtanteil des Rückfettersystems aus Isopropylpalmitate, Myristylalkohol und 2-Octyldodecanol und optional weiteren Verbindungen mit rückfettenden Eigenschaften 2,0 bis 3,5 Gew.-% beträgt, ist es bevorzugt, dass Isopropylpalmitate, Myristylalkohol und 2-Octyldodecanol jeweils zu mindestens 0,5 Gew.-% in dem erfindungsgemäßen Hygienegel enthalten sind.

Ein in dem erfindungsgemäßen Hygienegel bevorzugt enthaltenes Feuchthaltemittel ist Glycerin, dessen Gehalt maßgeblich verantwortlich für die hautpflegenden Eigenschaften des Hygienegels ist. Während aus dem Stand der Technik, wie beispielsweise in der WO 2018/015182 A1 erwähnt, bekannt ist, dass Glycerin die antimikrobiellen Eigenschaften einer Zusammensetzung abschwächen soll, so wurde im Rahmen der vorliegenden Erfindung gefunden, dass der Einsatz einer Kombination von Glycerin, dem Rückfettersystem aus Isopropylpalmitate, Myristylalkohol und 2-Octyldodecanol und dem im Vergleich zu klassischen Desinfektionsmitteln geringen Alkoholgehalt ein Hygienegel mit hoher antimikrobieller Wirksamkeit und hautpflegendem Effekt liefert.

Gemäß einer besonders bevorzugten Ausführungsform umfasst das erfindungsgemäße Hygienegel daher Glycerin, Isopropylpalmitat, 2-Octyldodecanol, Myristylalkohol, Wasser sowie einen oder mehrere Alkohole mit 1 bis 4 Kohlenstoffatomen, vorzugsweise Ethanol, oder besteht daraus.

Optional enthält das erfindungsgemäße Hygienegel darüber hinaus auch einen oder mehrere Verdicker, beispielsweise Xanthan Gum und/oder Konjac Gum. Als weitere mögliche Inhaltsstoffe können Filmbildner, z.B. Amorphophallus Konjac Root Extract, Konservierungsmittel, z.B. Zitronensäure, und/oder Parfum in dem erfindungsgemäßen Hygienegel enthalten sein.

Weiter optional enthält die Zusammensetzung auch zusätzliche Hautpflegemittel, wie z.B. Harnstoff, Dexpanthenol, Hamamelis, Bisabolol und/oder Allantoin, wobei diese zusätzlichen Hautpflegemittel insbesondere einen Gehalt von bis zu 0,9 Gew.-% in dem erfindungsgemäßen Hygienegel aufweisen.

Die Erfindung betrifft weiterhin auch ein Verfahren zur Reinigung von Haut, wobei die Haut mit dem erfindungsgemäßen Hygienegel kontaktiert wird, das vorzugsweise durch Verreiben bis zur Trocknungszeit auf der Hautoberfläche verteilt wird. Aufgrund der guten hautpflegenden Wirkung und antimikrobiellen Wirksamkeit ist das erfindungsgemäße Hygienegel dabei insbesondere für den Einsatz im privaten Bereich, beispielsweise im Rahmen der Baby- und Kleinkindpflege geeignet.

Entsprechend betrifft die Erfindung weiterhin auch die Verwendung des erfindungsgemäßen Hygienegels zur Reinigung von Haut, insbesondere von Händen.

### Genaue Beschreibung der Erfindung

Die Erfindung wird nun genauer anhand von Ausführungsbeispielen beschrieben.

### Beispiel 1: Herstellung eines erfindungsgemäßen Hygienegels mit hautpflegender und antimikrobieller Wirkung

Für die Herstellung eines ersten erfindungsgemäßen Hygienegels mit hautpflegender und antimikrobieller Wirkung wurden die folgenden in Tabelle 1 genannten Inhaltsstoffe zu den ebenfalls angegebenen Gewichtsanteilen zu einer homogenen flüssigen Zusammensetzung (Hygienegel A) vermischt.

**Tab. 1: Inhaltsstoffe des erfindungsgemäßen Hygienegels (Hygienegel A)**

| **Inhaltsstoff** | **Anteil /[Gew.-%]** |
|---|---|
| Wasser | Ad 100 |
| Glycerin | 0,70 |
| Isopropylpalmitate | 1,00 |
| Myristylalkohol | 1,00 |
| 2-Octyldodecanol | 0,80 |
| Alkohol denat. | 10,00 - 20,00 |
| Xanthan Gum | 1,0 |
| Amorphophallus Konjac Root Extract | 0,1 |
| Zitronensäure Monohydrat | 0,05 |

Für die Herstellung eines zweiten erfindungsgemäßen Hygienegels mit hautpflegender und antimikrobieller Wirkung wurden die folgenden in Tabelle 2 genannten Inhaltsstoffe zu den ebenfalls angegebenen Gewichtsanteilen zu einer homogenen flüssigen Zusammensetzung (Hygienegel B) vermischt.

**Tab. 2: Inhaltsstoffe des erfindungsgemäßen Hygienegels (Hygienegel B)**

| **Inhaltsstoff** | **Anteil /[Gew.-%]** |
|---|---|
| Wasser | Ad 100 |
| Glycerin | 0,70 |
| Isopropylpalmitate | 1,00 |
| Myristylalkohol | 1,00 |
| 2-Octyldodecanol | 0,80 |
| Alkohol denat. | 10,00 - 20,00 |
| Xanthan Gum | 1,0 |
| Amorphophallus Konjac Root Extract | 0,1 |
| Zitronensäure Monohydrat | 0,05 |
| Parfum | 0,1 |

Für die Herstellung eines dritten erfindungsgemäßen Hygienegels mit hautpflegender und antimikrobieller Wirkung, das zusätzlich zu dem erfindungsgemäßen Rückfettersystem noch Dicaprylyl Ether als weiteren Rückfetter enthielt, wurden die folgenden in Tabelle 3 genannten Inhaltsstoffe zu den ebenfalls angegebenen Gewichtsanteilen zu einer homogenen flüssigen Zusammensetzung (Hygienegel C) vermischt.

**Tab. 3: Inhaltsstoffe des erfindungsgemäßen Hygienegels (Hygienegel C)**

| **Inhaltsstoff** | **Anteil /[Gew.-%]** |
|---|---|
| Wasser | Ad 100 |
| Glycerin | 0,70 |
| Isopropylpalmitate | 1,00 |
| Myristylalkohol | 1,00 |
| 2-Octyldodecanol | 0,80 |
| Dicaprylyl Ether | 1,00 |
| Ethanol 99%ig vergällt 1% MEK | 20,00 |
| Xanthan Gum | 1,0 |
| Konjac Gum | 0,1 |
| Zitronensäure Monohydrat | 0,05 |

Die Herstellung aller drei Hygienegele A, B und C erfolgte dabei, indem zunächst jeweils zwei Phasen A und B hergestellt wurden. Phase A enthielt dabei jeweils Wasser und Verdicker, d.h. im Fall von Hygienegel A und B Xanthan Gum sowie im Fall von Hygienegel C Xanthan Gum und Konjac Gum. In allen drei Fällen wurde der pH-Wert der Phase A mit Zitronensäure auf pH 7 eingestellt.

Phase B, die aus den übrigen Bestandteilen bestand, wurde anschließend in Phase A eingerührt, wobei mit fortschreitender Zeit immer stärker gerührt werden musste. Mit Zitronensäure wurde ein pH-Wert zwischen 5 und 6 eingestellt. Anschließend wurde unter Rühren homogenisiert und in allen drei Fällen wurde ein emulsionsartiges Gel erhalten.

Von allen auf diese Weise hergestellten Hygienegelen wurde die Viskosität bestimmt. Diese betrug bei dem Hygienegel A 220 mPa·s, bei dem Hygienegel B 150 mPa·s und bei dem Hygienegel C ebenfalls 150 mPa·s, jeweils gemessen mit einem DIN-Messsystem 33 gemäß ISO 53019 bei einer Temperatur von 293,15 K und mit einer Umdrehungszahl von 500 r/min.

Alle drei erfindungsgemäßen Hygienegele zeichnen sich durch eine gute antimikrobielle Wirksamkeit, eine gute Handhabbarkeit beim Auftragen auf die Haut (durch die gelartige Konsistenz) sowie durch ein erzeugtes angenehmes Hautgefühl aus.

Die in der vorstehenden Beschreibung, in den Beispielen und in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Hygienegel mit hautpflegendem Effekt und antimikrobieller Wirksamkeit, enthaltend
- 5 bis 35 Gew.-% Alkohol, wobei der Alkohol 1 bis 4 Kohlenstoffatome aufweist;
- zumindest 0,3 Gew.-% Feuchthaltemittel, wobei das Feuchthaltemittel aus der Gruppe ausgewählt ist, die Glycerin, Propylenglycol, Butan-1,2-diol, Butan-1,3-diol und Kombinationen dieser umfasst, und
- ein Rückfettersystem, das Isopropylpalmitate, Myristylalkohol und 2-Octyldodecanol als Verbindungen mit rückfettenden Eigenschaften enthält,
wobei das Hygienegel eine Viskosität von 115 mPa·s bis 450 mPa·s, gemessen mit einem DIN-Messsystem 33 gemäß ISO 53019 bei einer Temperatur von 293,15 K und mit einer Umdrehungszahl von 500 r/min, aufweist.

2. Hygienegel nach Anspruch 1, **dadurch gekennzeichnet, dass** es zumindest eine weitere Verbindung mit rückfettenden Eigenschaften enthält, die aus der Gruppe ausgewählt ist, die PEG-7 Glyceryl Cocoate, Lanolin, Dicaprylyl Ether, Sorbitan Sesquicaprylate, Isopropylmyristate, Polyglyceryl-4-Caprate, Capryl/Caprinsäure Triglycerid (Caprylic/Capric Triglyceride), Cetearyl Ethylhexanoate, Glyceryl Laurate und Kombinationen dieser umfasst.

3. Hygienegel nach einem der Ansprüche 1 oder 2, **dadurch gekennzeichnet, dass** der Anteil an Alkohol 10 bis 30 Gew.-% und insbesondere 10 bis 25 Gew.-% beträgt.

4. Hygienegel nach einem der voranstehenden Ansprüche, **dadurch gekennzeichnet, dass** der Alkohol aus der Gruppe ausgewählt ist, die Methanol, Ethanol, Propanol, Isopropanol, Butanol, Isobutanol und Mischungen dieser umfasst.

5. Hygienegel nach einem der voranstehenden Ansprüche, **dadurch gekennzeichnet, dass** der Anteil an Feuchthaltemittel an der Zusammensetzung 0,5-3,5 Gew.-% beträgt.

6. Hygienegel nach einem der voranstehenden Ansprüche, **gekennzeichnet durch** eine Viskosität von 200 mPa·s bis 400 mPa·s, gemessen mit einem DIN-Messsystem 33 gemäß ISO 53019 bei einer Temperatur von 293,15 K und mit einer Umdrehungszahl von 500 r/min.

7. Hygienegel nach einem der voranstehenden Ansprüche, **dadurch gekennzeichnet, dass** das Feuchthaltemittel Glycerin ist.

8. Hygienegel nach einem der voranstehenden Ansprüche, **dadurch gekennzeichnet, dass** dieses Glycerin, Isopropylpalmitat, 2-Octyldodecanol, Myristylalkohol, Wasser sowie einen oder mehrere Alkohole mit 1 bis 4 Kohlenstoffatomen umfasst oder daraus besteht.

9. Verfahren zur Reinigung von Haut, **dadurch gekennzeichnet, dass** die Haut mit einem Hygienegel nach einem der Ansprüche 1 bis 8 kontaktiert wird.

10. Verwendung eines Hygienegels nach einem der Ansprüche 1 bis 8 zur Reinigung von Haut.

## Claims

1. Hygienic gel having skin-care effect and antimicrobial activity, containing
- 5 to 35% by weight of alcohol, wherein the alcohol has 1 to 4 carbon atoms;
- at least 0.3% by weight of humectant, wherein the humectant is selected from the group comprising glycerin, propylene glycol, butane-1,2-diol, butane-1,3-diol, and combinations thereof, and
- a refatting system containing isopropyl palmitate, myristyl alcohol, and 2-octyldodecanol as compounds having refatting properties,
wherein the hygienic gel has a viscosity of 115 mPa·s to 450 mPa·s, measured using a DIN measuring system 33 according to ISO 53019 at a temperature of 293.15 K and at a rotation speed of 500 r/min.

2. Hygienic gel according to claim 1, **characterized in that** it contains at least one further compound having refatting properties selected from the group comprising PEG-7 glyceryl cocoate, lanolin, dicaprylyl ether, sorbitan sesquicaprylate, isopropyl myristate, polyglyceryl-4 caprate, caprylic/capric acid triglyceride (caprylic/capric triglyceride), cetearyl ethylhexanoate, glyceryl laurate and combinations thereof.

3. Hygienic gel according to one of claims 1 or 2, **characterized in that** the alcohol content is 10 to 30% by weight and, in particular, 10 to 25% by weight.

4. Hygienic gel according to one of the preceding claims, **characterized in that** the alcohol is selected from the group comprising methanol, ethanol, propanol, isopropanol, butanol, isobutanol, and mixtures thereof.

5. Hygienic gel according to one of the preceding claims, **characterized in that** the proportion of humectant in the composition is 0.5-3.5% by weight.

6. Hygienic gel according to one of the preceding claims, **characterized by** a viscosity of 200 mPa·s to 400 mPa·s, measured using a DIN measuring system 33 according to ISO 53019 at a temperature of 293.15 K and at a rotation speed of 500 r/min.

7. Hygienic gel according to one of the preceding claims, **characterized in that** the humectant is glycerin.

8. Hygienic gel according to one of the preceding claims, **characterized in that** it comprises or consists of glycerin, isopropyl palmitate, 2-octyldodecanol, myristyl alcohol, water, and one or more alcohols with 1 to 4 carbon atoms.

9. Process for cleaning skin, **characterized in that** the skin is contacted with a hygienic gel according to one of claims 1 to 8.

10. Use of a hygienic gel according to one of claims 1 to 8 for cleaning of skin.

## Revendications

1. Gel d'hygiène à effet de soin dermatologique et activité antimicrobienne, contenant
- 5 à 35 % en poids d'alcool, l'alcool ayant de 1 à 4 atomes de carbone ;
- au moins 0,3 % en poids d'humectant, l'humectant étant choisi dans le groupe qui comprend le glycérol, le propylèneglycol, le butane-1,2-diol, le butane-1,3-diol et des associations de ceux-ci, et
- un système de regraissage qui contient en tant que composés ayant des propriétés de regraissage du palmitate d'isopropyle, de l'alcool myristylique et du 2-octyldodécanol,
le gel d'hygiène présentant une viscosité de 115 mPa·s à 450 mPa·s, mesurée au moyen d'un système de mesure DIN 33 selon ISO 53019 à une température de 293,15 K et à une vitesse de rotation de 500 tours/min.

2. Gel d'hygiène selon la revendication 1, **caractérisé en ce qu'**il contient au moins un composé supplémentaire ayant des propriétés de regraissage, qui est choisi dans le groupe qui comprend le PEG-7 Glyceryl Cocoate, la lanoline, l'éther dicaprylylique, le sesquicaprylate de sorbitane, le myristate d'isopropyle, le Polyglyceryl-4-Caprate, le triglycéride d'acide caprylique/acide caprique (Caprylic/Capric Triglyceride), l'éthylhexanoate de cétéaryle, le laurate de glycéryle et des associations de ceux-ci.

3. Gel d'hygiène selon l'une quelconque des revendications 1 ou 2, **caractérisé en ce que** la teneur en alcool vaut de 10 à 30 % en poids et en particulier 10 à 25 % en poids.

4. Gel d'hygiène selon l'une quelconque des revendications précédentes, **caractérisé en ce que** l'alcool est choisi dans le groupe qui comprend le méthanol, l'éthanol, le propanol, l'isopropanol, le butanol, l'isobutanol et des mélanges de ceux-ci.

5. Gel d'hygiène selon l'une quelconque des revendications précédentes, **caractérisé en ce que** la teneur en humectant de la composition vaut 0,5-3,5 % en poids.

6. Gel d'hygiène selon l'une quelconque des revendications précédentes, **caractérisé par** une viscosité de 200 mPa·s à 400 mPa·s, mesurée au moyen d'un système de mesure DIN 33 selon ISO 53019 à une température de 293,15 K et à une vitesse de rotation de 500 tours/min.

7. Gel d'hygiène selon l'une quelconque des revendications précédentes, **caractérisé en ce que** l'humectant est le glycérol.

8. Gel d'hygiène selon l'une quelconque des revendications précédentes, **caractérisé en ce qu'**il comprend du glycérol, du palmitate d'isopropyle, du 2-octyldodécanol, de l'alcool myristylique, de l'eau ainsi qu'un ou plusieurs alcools ayant de 1 à 4 atomes de carbone ou est constitué de ceux-ci.

9. Procédé pour le nettoyage de la peau, **caractérisé en ce que** la peau est mise en contact avec un gel d'hygiène selon l'une quelconque des revendications 1 à 8.

10. Utilisation d'un gel d'hygiène selon l'une quelconque des revendications 1 à 8 pour le nettoyage de la peau.
